# EUROPEAN PATENT SPECIFICATION

(11) **EP 3 592 975 B1**
(45) Date of publication and mention of the grant of the patent: **07.05.2025**
(21) Application number: 18764967.8
(22) Date of filing: 08.03.2018
(51) Int. Cl.: F04B 39/00, F04B 37/14, A61M 1/00, A61M 1/06

(54) **VENTING DEVICE SYSTEM AND METHOD OF VENTING WITHIN A CHAMBER OF A RECIPROCATING DEVICE ASSEMBLY**
ENTLÜFTERSYSTEM UND ENTLÜFTUNGSVERFAHREN IN EINER KAMMER EINER HUBKOLBENANORDNUNG
SYSTÈME DE DISPOSITIF DE MISE À L'AIR LIBRE ET PROCÉDÉ DE MISE À L'AIR LIBRE DANS UNE CHAMBRE D'UN ENSEMBLE DISPOSITIF À VA-ET-VIENT

(30) Priority: 10.03.2017 US 201762469625 P
(43) Date of publication of application: 15.01.2020
(73) Proprietor: Medela Holding AG, 6340 Baar (CH)
(72) Inventor: WACH, Joseph, McHenry, Illinois 60050 (US); STEVENS, Natalie, McHenry, Illinois 60050 (US)
(74) Representative: Grünecker Patent- und Rechtsanwälte PartG mbB
(86) International application number: PCT/US2018/021490
(87) International publication number: WO 2018/165393

(56) References cited:
- EP-A1- 0 094 231
- US-A- 2 222 811
- US-A- 2 915 986
- US-A- 3 367 278
- US-A- 4 287 819
- US-A- 4 583 970
- US-A- 4 583 970
- US-A- 5 449 278
- US-A- 5 494 410

## Description

### FIELD OF THE DISCLOSURE

The present disclosure relates generally to a venting device system and method for venting a reciprocating device assembly.

### BACKGROUND

Reciprocating device assemblies can include one or more fixed pathways to vent fluid within a reciprocating device chamber at a specific point during operation of the reciprocating device. The pathways generally address unwanted pressure generated in the chamber during operation of the reciprocating device.

US Patent No. 9,080,710; and 5,735,314 each address various pathways incorporated into a piston assembly to vent the chamber during operation. For example, US Patent No. 9,080,710 discusses an accumulator reservoir including a multitude of paths that can and will operate in parallel and provide an interconnected network of vent flows. US patent 5,735,314 discusses a dampening diaphragm enclosing a damping chamber into which a throttle bore discharges through the housing end wall.

US Patent No. 2,222,811 discusses a breast pump assembly having a disc-like flanged sealing ring clamped to a top of a piston head by a metallic disc. The flanged sealing ring is formed by a leather disc having an out-turned flange that surrounds an endless spiral spring, and by the natural expansion of the latter, is yieldingly held in contact with the walls of the cylinder. Under upward or inward movement of the piston, air caged in the cylinder will find relatively free escape therefrom past the flexible flange of the piston ring, especially at or in line with the flattened portion. The device works without the use of any check valve in any of the air passages to or from the cylinder. However, additional space is required within the cylinder to accommodate the height of the flanged sealing ring above the piston.

US Patent No. 4,583,970 relates to a milk suction device with a funnel and a piston pump connected thereto. The device has a hollow piston rod with a return valve having an opening located in the suction space of a cylinder of the pump. The return valve is closed during the suction stroke of the piston pump and is constructed as an aeration valve.

European patent application EP 0 094 231 A1 relates to a nebulizer that has a nebulizing head and is supplied with compressed air from a pump which is powered by the exertions of an individual, who may be the user. The pump incorporates a cylinder with a reciprocable piston which generates compressed air on one stroke but not on the reverse stroke. An accumulator receives compressed air from the pump and enables the head to produce a continuous spray. The accumulator may include a fine filter. The pump may resemble a foot-pump for inflating vehicle tyres.

US Patent application No. 5 494 410 A relates to a manually operable vacuum pump having a housing including first and second pump chambers whose volume is altered by movement of a displaceable piston which is moved by means of a piston rod attached thereto and fed through one of the first and second pump chambers. The first and second pump chambers are configured such that gas which is fed into the vacuum pump is compressed in two steps, primarily in the first pump chamber and secondarily in the second pump chamber to provide a suitable end vacuum. Thereafter, the gas is expelled from the second pump chamber through the second end cap.

US Patent application No. 5 449 278 A relates to a double action piston having plural annular check valves. An air pump piston is provided peripherally with a first leak proof ring and a second leak proof ring, which are located respectively and contiguously at both ends of the piston. Located peripherally between the first and the second leak proof rings is a radially disposed air hole. The piston is fastened at the second end thereof with a piston rod such that the air hole of the piston is in communication with an axial passageway of the piston rod. The piston is further provided peripherally between the first leakproof ring and the air hole with at least one first duct disposed radially. The piston is still further provided peripherally with at least one second duct which is disposed radially and located between the second leak proof ring and the air hole.

### SUMMARY

It is an object of the present invention to provide a venting device system, a breast pump assembly and a method of reducing unwanted pressure in a reciprocating device assembly that obviate or mitigate at least one of the disadvantages and shortcomings of the related prior art. This object is solved by the present invention as claimed in the appended independent claims. Particular embodiments of the present invention are defined by the appended dependent claims.

In accordance with the principles of the present disclosure, a venting device system of a reciprocating device assembly that eliminates the disadvantages of the previous systems, is set forth. A more compact and efficient assembly can thus be achieved in accordance with the principles herein. Moreover, a system configured to provide an improved flow path for venting a reciprocating device assembly is provided, in accordance with the principles herein.

A wide variety of embodiments are contemplated, and can be constructed in accordance with the principles herein to provide an improved flow path via an improved venting device system while generating fluid pressure in the system.

Although exemplary embodiments are set forth to illustrate the basic advantages of the improvements achieved in accordance with the principles herein, numerous other embodiments are contemplated that achieve a flow path that does not require either structure positioned above a head of the reciprocating device during operation or a fixed vent. For example, in one exemplary embodiment the flow path can vary due to movement of a component, such as a seal or sealing member. The component can move a venting device system, from an open vent, during a return stroke of a reciprocating device, to a closed vent, during a pressure generating stroke of the reciprocating device. The venting device system can be configured in any suitable arrangement to include at least one selectively sealing component, or seal that can be movable or bendable during operation of the reciprocating device to form a variable air flow path, while moving through the reciprocating device assembly. For example, the venting device system can be configured to include a seal that can move and seal the reciprocating device during a pressure generating stroke of the reciprocating device. In an exemplary embodiment, the venting device system can be configured to include a seal that travels back and forth relative to the reciprocating piston assembly head components during operation, where the venting can be tuned based on movement of the seal relative to the reciprocating device, and/or other parameters of the reciprocating device assembly. As a result the venting device system can improve the efficiency of the movement of the reciprocating device by improving the distribution of fluid within a chamber of the reciprocating device assembly during operation.

In yet another exemplary embodiment, the venting device system can include a deformable seal, incorporated in any suitable manner, into the design of the reciprocating device, for example by integrally forming the seal with a head of the reciprocating device, so that the function of the venting device system is an integral part of the reciprocating device. In other exemplary embodiments, the venting device system can include a seal partially formed in the reciprocating device and partially formed as a separate or connectable component to the reciprocating device. In still other exemplary embodiments, the venting device system can include a seal that moves relative to a sealing section of a reciprocating device, such that no direct connection exits continually between the reciprocating device and the seal during operation. Suitable variations and combinations of the examples set forth herein are contemplated as well.

In accordance with the principles of the present disclosure one exemplary embodiment can include a breast pump assembly having a venting device system including a seal selectively connectable to or integrally formed in a head of a reciprocating device. The seal of the venting device system can be disposed below the top of the head of the reciprocating device. The seal of the venting device system can be configured to selectively move, based on forces present in the system during operation of the reciprocating device.

In an exemplary breast pump assembly, the venting device system can be configured to seal the reciprocating device during a pressure generating stroke and to vent the assembly during a return stroke. The breast pump assembly can further include a chamber, wherein the venting device system further eliminates unwanted pressure in the chamber of the assembly during operation without affecting pressure creation in the chamber during a pressure generating stroke of the assembly. The venting device system can be configured of one or more materials, including a deformable material and/or a sealable material.

In yet another embodiment constructed in accordance with the principles herein a medical device assembly can include a venting device system configured to move based on forces present in the assembly. The venting device system can be further configured to include a seal adapted to selectively engage a portion of a head of a reciprocating device below the top of the reciprocating device on a pressure generating stroke of the reciprocating device to substantially seal the reciprocating device within a chamber of the assembly.

In still another embodiment the venting device system can include a seal that movingly engages different parts of the head of the reciprocating device. The venting device system can be suitably disposed on, around or near the reciprocating member. For example, in an embodiment the venting device system can include a seal movably seated about an interior of a head of the reciprocating member. The venting device system can be configured to allow fluid to flow in an opposite direction from a direction of travel of a reciprocating device, given movement of the reciprocating device on a return stroke. The venting device system can be configured to include a seal that at least partially moves into a position to seal the reciprocating device during a pressure generating stroke of the reciprocating device.

An exemplary method of reducing unwanted pressure in a venting device system within a reciprocating device assembly can include the following steps: venting air from above a reciprocating device around a top of the reciprocating device on a return stroke with a seal disposed below the top of the reciprocating device; and sealing air below the top of the reciprocating device on a pressure generating stroke with the seal of the venting device system on a pressure generating stroke. The method can further include the step of configuring the seal of the venting device system to move from a first position substantially near the top of the reciprocating member on a pressure generating stroke to a second position displaced away from the first position, and disposed directly or indirectly against a surface of the reciprocating device on a return stroke.

Various advantages of the present disclosure are specifically described below in reference to the exemplary embodiments, or conceptually embodied therein. The drawings and description herein are provided to merely illustrate examples of the general concepts discussed throughout the present disclosure. Numerous changes and modifications can be made, as known to those of skill in the art, without departing from the scope of the invention as defined by the appended claims.

### BRIEF DESCRIPTION OF THE DRAWINGS

These and other features and advantages of the various exemplary embodiments disclosed herein will be better understood with respect to the following description and drawings, in which:
Figure 1 is a view, partially in section, of a venting device system including a reciprocating device assembly and a seal during a return stroke of a reciprocating device;
Figure 2 is a view, partially in section, of a venting device system including a reciprocating device assembly and a seal during a pressure generating stroke of a reciprocating device;
Figure 3 is a top view of an exemplary embodiment of a customized seal for a venting device system constructed in accordance with the principles herein;
Figure 4 is a side view of the exemplary seal of Figure 3;
Figure 5 is a bottom view of the exemplary seal of Figure 3;
Figure 6 is a front sectional view taken along lines VI-VI of the exemplary seal of Figure 3;
Figures 7 and 8 are exemplary embodiments of exemplary venting device system components including custom features to fine tune fluid flow in the system; and
Figure 9 is another embodiment of a venting device system including a magnetic drive mechanism formed of at least one magnet. Common reference numerals are used throughout the drawings and the detailed description to indicate the same elements.

### DETAILED DESCRIPTION

The detailed description set forth below in connection with the appended drawings is intended as a description of certain exemplary embodiments of various system components constructed in accordance with the principles herein. These examples are not intended to represent the only embodiments or forms that may be developed or utilized according to these principles. It is further understood that the use of relational terms such as first and second, and the like are used solely to distinguish one entity from another without necessarily requiring or implying any actual such relationship or order between such entities.

Certain aspects of some embodiments constructed in accordance with the principles herein are directed toward a reciprocating device assembly having improved venting efficiency during operation.

It is understood that the systems described herein may be used to deliver a wide range of fluid contents to a wide variety of reciprocating device assemblies for delivering positive or negative pressure. Such contents will be collectively referred to herein as "fluid" for purposes of simplicity.

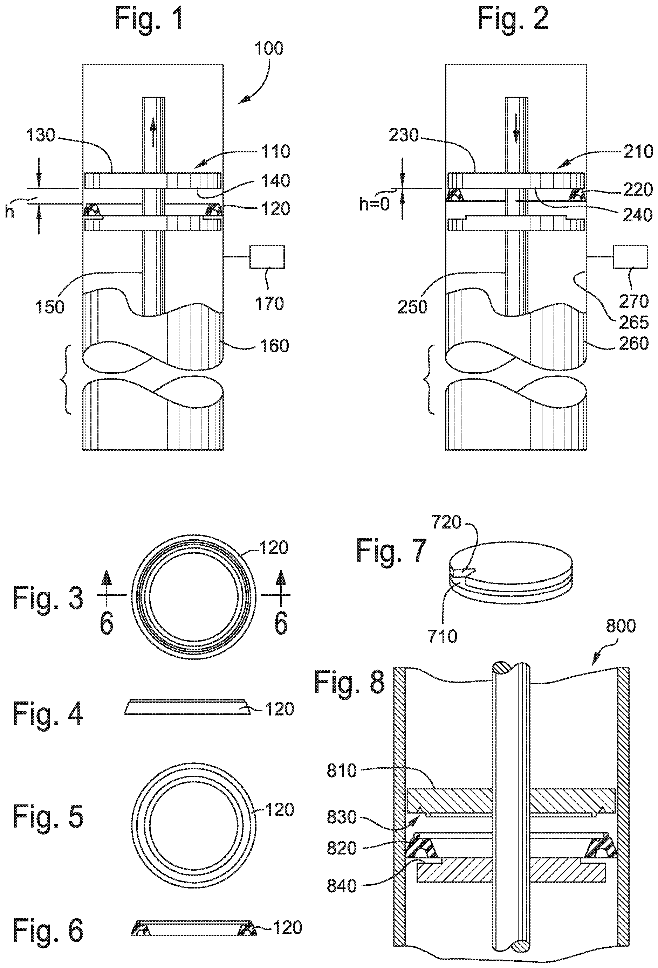

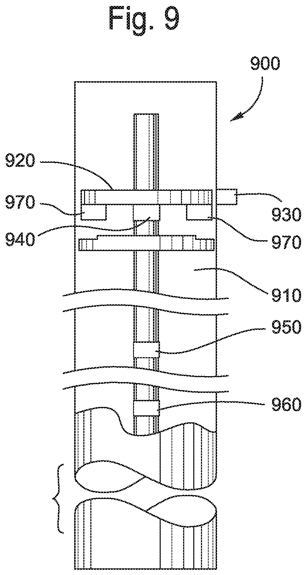

An exemplary embodiment of a venting device system including a reciprocating device assembly constructed in accordance with the principles herein is shown generally at 100 in Figure 1. A suitable reciprocating device 110 can be provided and the venting device system 100 can further include a seal 120 configured and arranged within the reciprocating device assembly 110 to seat in a first position A relative to the reciprocating device 110 during a return stroke of the reciprocating device 110 (in this example the reciprocating device assembly is oriented in an upright position, although numerous other orientations are contemplated and are within the scope of the present disclosure). In position A, the seal 120 provides a fluid path of a preselected height h, (shown here between a top of the seal 120 and a lower surface of a head 140 of the reciprocating device 110), determined to best meet the needs of a particular system. Thus, during the return stroke the seal 120 provides a fluid path that vents fluid from one side of the piston to an opposite position 140 in the reciprocating device assembly 110, illustrated here as disposed below the head 140 of the piston. Thus, Figure 1 shows an example of how a system constructed in accordance with the principles herein can provide a desired fluid flow path without requiring a fixed vent or an enlarged structure. The reciprocating device assembly 110 can further include a stem 150 for moving the head 140 back and forth in a chamber 160 of the venting device system. A suitable motor assembly 170 can be provided and connected either directly or indirectly to the chamber 160 and/or stem 150 to facilitate movement of the stem 150, if desired. One example of a suitable motor for certain applications is a gear motor. Other force driving motors or devices can be provided and selected based on desired system performance or to meet specific requirements, for example a piston driving device as described in Figure 9 below, or any other suitable device. The system illustrated in Figure 1 is a negative pressure generating system. Other systems, such as positive generating pressure systems, are contemplated herein.

Figure 2 illustrates an exemplary embodiment of a venting device system shown generally at 200 and including a reciprocating device 210 and a seal 220. The seal 220 is shown seated in a different position relative to a reciprocating device assembly 210 compared to the position shown in Figure 1 during a pressure generating stroke of the system 200. As the direction of the reciprocating device turns so that the stem 250 moves due to the driving force of a suitable device or assembly 270 for the pressure generating stroke, the seal 220 can be configured to move, by the forces in the system via 270 or by any other suitable force or combination of forces, to a different position from the Position A of Figure 1 to a Position B in Figure 2. To this end, any suitable shape or materials can be used to form the seal 220, so long as the device seals the system during the pressure generation stroke. Additionally, an off the shelf seal may be suitable for certain applications. Any suitable material or combination of materials can be used to form the seal 220, including an elastic material, so long as the material can be configured to seal the system at the cylinder and the piston. From the Position B the seal 220 can expand toward an interior wall 265 of a chamber 260 of the venting device system 200 and substantially or fully seal the fluid above or below a head 240 (depending on whether the system is creating vacuum or pressure) of the reciprocating device 210 during the pressure generating stroke. As a result, a highly efficient pressure performance can be achieved in accordance with the principles herein.

Venting device systems constructed in accordance with the principles herein can include a selectively connectable sealing member in association with a reciprocating device assembly, that selectively seals the fluid in the chamber, either directly or indirectly as the seal in the system can be configured to float, either partially or fully, from position A to position B, or from a non-sealing position to a sealing position. A stem can be provided for moving the reciprocating device in a chamber of the system. The stem can be powered by any suitable device or configuration, as desired, to achieve output requirements of the reciprocating device assembly for a chosen system. The stem can be connectable to a suitable driving component of the system.

Figures 3 through 6 illustrate various views of an exemplary embodiment of a custom seal of a venting device system constructed in accordance with the principles herein. Figure 3 illustrates a top view, Figure 4 illustrates a side view, Figure 5 illustrates a bottom view and Figures 6 illustrates front/back sectional views taken along VI-VI of Figure 3.

Figure 7 illustrates yet another exemplary embodiment including customized components constructed for use in a venting device system, wherein one or more customizable features can be provided, as desired, in order to fine tune the venting requirements of a particular system. Such customizable features can include, for example, a groove in a vented piston head 720, and at least one deformable seal portion 710. In this exemplary embodiment the seal portion 710 only partially moves in response to movement of the reciprocating member head 720. In yet another exemplary embodiment, shown in Figure 8, a rib or feature to seal and prevent leaking can be incorporated into an embodiment of a venting system 800 constructed in accordance with the principles herein. For example, a reciprocating head 810 can include a sealing feature, such as sealing feature 830, which can also provide a tortuous path on the sealing surface when mated with a custom seal 820 (also illustrated in Figures 3 to 6 above). The top of seal 820 and the reciprocating head 810 can be customized with any number of a variety of geometries in accordance with the needs of a given system. Additionally, any suitable geometry 840 can be selected in a lower head of the piston to help facilitate a desired air flow path.

As illustrated in Figure 9, a magnetically-driven venting device system, shown generally at 900, can include a chamber 910 and one or more magnets 930 to 960 selected to cooperate to move a reciprocating member 920. Here, one or more seals 970 can be incorporated into the system, as desired, to accommodate the requirements for a particular system. Many other embodiments are contemplated herein, and include customizing the pressure generating component of the system to improve fluid flow to vent the system during a return stroke while maintaining a compact pressure generating chamber, without directing the fluid through a head of the pressure generating component.

Exemplary systems wherein the principles herein can provide the aforementioned advantages include, but are not limited to, breast pump assemblies, wound drainage and therapy devices, and other systems including reciprocating devices assemblies.

## Claims

1. A venting device system configured to allow fluid to flow in opposite direction of a reciprocating device (110; 210) given movement of the reciprocating device (110; 210) on a return stroke, wherein the reciprocating device (110; 210) is a piston and includes a head (140; 240; 810) which is movable back and forth in a chamber (160; 260),
the venting device system including a seal (120; 220; 820) configured and arranged to seat in a first position (A), below the head (140; 240; 810) of the reciprocating device (110; 210), during a return stroke of the reciprocating device (110; 210), wherein in the first position (A), the seal (120; 220; 820) provides a fluid path of a preselected height (h) between a top of the seal (120; 220; 820) and a lower surface of the head (140; 240; 810) of the reciprocating device (110; 210), and to seat in a second position (B), different from the first position (A), during a pressure generating stroke;
wherein the seal (120; 220; 820) is made from an elastic material and is further configured to at least partially move into the second position (B) and to expand, from the second position (B), toward an interior wall (265) of the chamber (160; 260) to substantially or fully seal the fluid below the head (140; 240; 810) of the reciprocating device (110; 210) during the pressure generating stroke of the reciprocating device (110; 210).

2. The venting device system of claim 1, wherein the venting device system includes a vented piston head (720) with a groove and at least one deformable seal portion (710) which only partially moves in response to movement of the vented piston head (720).

3. A breast pump assembly comprising:
a venting device system (100; 200; 800) according to claim 1,
wherein the seal (120; 220; 820) is selectively connectable to the head of the reciprocating device (110; 210), and is configured to selectively move, based on forces present in the system during operation of the reciprocating device (110; 210).

4. The breast pump assembly of claim 3, the venting device system is configured to seal the reciprocating device (110; 210) during a pressure generating stroke and to vent the assembly during a return stroke.

5. The breast pump assembly of claim 3, further comprising a chamber (160), wherein the venting device system further eliminates unwanted pressure in the chamber (160) of the assembly during operation without affecting pressure creation in the chamber (160) during a pressure generating stroke of the assembly.

6. The breast pump assembly of claim 3, the venting device system is further configured of a sealable material.

7. A medical device assembly comprising:
a venting device system (100; 200; 800) according to claim 1,
wherein the seal (120; 220; 820) of the venting device system (100; 200; 800) is configured to move based on forces present in the assembly and further configured to selectively engage a portion of the head of the reciprocating device (110; 210), below the top of the reciprocating device (110; 210) on a pressure generating stroke of the reciprocating device (110) to substantially seal the reciprocating device (110; 210) within a chamber (160) of the assembly.

8. The medical device assembly of claim 7, wherein the venting device system (100; 200; 800) movingly engages different parts of the head (140) of the reciprocating device (110; 210).

9. The medical device assembly of claim 7, the venting device system (100; 200; 800) movably seated about the head (140; 240; 810) of the reciprocating device (110; 210).

10. A method of reducing unwanted pressure in a reciprocating device assembly comprising the following steps:
venting air from above a reciprocating device (110; 210) around a head (140; 240; 810) of the reciprocating device (110; 210) on a return stroke with a venting device system (100; 200; 800) according to claim 1, the seal (120; 220; 820) of the venting device system (100; 200; 800) being disposed below the head (140; 240; 810) of the reciprocating device (110; 210); and
sealing air below the head (140; 240; 810) of the reciprocating device (110; 210) on a pressure generating stroke with the seal (120; 220; 820) of the venting device system (100; 200; 800) on a pressure generating stroke.

11. The method of claim 10, further comprising the step of configuring the seal (120; 220; 820) of the venting device system (100; 200; 800) to move from the second position (B) substantially near the top (140; 240; 810) of the reciprocating device (110; 210) on the pressure generating stroke to the first position (A), displaced away from the second position (B), and disposed directly or indirectly against a lower surface of the reciprocating device (110; 210) on a return stroke.

## Patentansprüche

1. Entlüftungsvorrichtungssystem, ausgebildet zum Ermöglichen, dass Fluid in die entgegengesetzte Richtung einer Kolbenvorrichtung (110; 210) bei einer Bewegung der Kolbenvorrichtung (110; 210) auf einem Rückhub strömt, wobei die Kolbenvorrichtung (110; 210) ein Kolben ist und einen Boden (140; 240; 810) umfasst, der in einer Kammer (160; 260) vor- und zurückbewegbar ist,
wobei das Entlüftungsvorrichtungssystem eine Dichtung (120; 220; 820), ausgebildet und angeordnet zum Sitzen in einer ersten Position (A) unter dem Boden (140; 240; 810) der Kolbenvorrichtung (110; 210) während eines Rückhubs der Kolbenvorrichtung (110; 210), wobei die Dichtung (120; 220; 820) in der ersten Position (A) einen Fluidweg einer vorgewählten Höhe (h) zwischen einer Oberseite der Dichtung (120; 220; 820) und einer Unterseite des Bodens (140; 240; 810) der Kolbenvorrichtung (110; 210) bereitstellt, und zum Sitzen in einer zweiten Position (B), die sich von der ersten Position (A) unterscheidet, während eines Druckerzeugungshubs, umfasst;
wobei die Dichtung (120; 220; 820) aus einem elastischen Material hergestellt ist und ferner dazu ausgebildet ist, sich wenigstens teilweise in die zweite Position (B) zu bewegen und sich aus der zweiten Position (B) in Richtung einer Innenwand (265) der Kammer (160; 260) auszudehnen, um das Fluid unterhalb des Bodens (140; 240; 810) der Kolbenvorrichtung (110; 210) während des druckerzeugenden Hubs der Kolbenvorrichtung (110; 210) im Wesentlichen oder vollständig abzudichten.

2. Entlüftungsvorrichtungssystem nach Anspruch 1, wobei das Entlüftungsvorrichtungssystem einen entlüfteten Kolbenboden (720) mit einer Nut und mindestens einem verformbaren Dichtungsabschnitt (710) umfasst, der sich als Reaktion auf die Bewegung des entlüfteten Kolbenbodens (720) nur teilweise bewegt.

3. Eine Milchpumpenanordnung, umfassend:
ein Entlüftungsvorrichtungssystem (100; 200; 800) nach Anspruch 1,
wobei die Dichtung (120; 220; 820) selektiv mit dem Boden der Kolbenvorrichtung (110; 210) verbindbar und zum selektiven Bewegen basierend auf den im System während des Betriebs der Kolbenvorrichtung (110; 210) vorhandenen Kräften ausgebildet ist.

4. Milchpumpenanordnung nach Anspruch 3, wobei das Entlüftungsvorrichtungssystem zum Abdichten der Kolbenvorrichtung (110; 210) während eines druckerzeugenden Hubs und Entlüften der Anordnung während eines Rückhubs ausgebildet ist.

5. Milchpumpenanordnung nach Anspruch 3, ferner umfassend eine Kammer (160), wobei das Entlüftungsvorrichtungssystem ferner unerwünschten Druck in der Kammer (160) der Anordnung während des Betriebs beseitigt, ohne die Druckerzeugung in der Kammer (160) während eines druckerzeugenden Hubs der Anordnung zu beeinflussen.

6. Milchpumpenanordnung nach Anspruch 3, wobei das Entlüftungsvorrichtungssystem ferner aus einem abdichtbaren Material ausgebildet ist.

7. Medizinische Vorrichtungsanordnung, umfassend:
ein Entlüftungsvorrichtungssystem (100; 200; 800) nach Anspruch 1,
wobei die Dichtung (120; 220; 820) des Entlüftungsvorrichtungssystems (100; 200; 800) zum Bewegen basierend auf der in der Anordnung vorhandenen Kräfte ausgebildet ist und ferner zum selektiven Eingriff eines Abschnitts des Bodens der Kolbenvorrichtung (110; 210) unterhalb der Oberseite der Kolbenvorrichtung (110; 210) bei einem druckerzeugenden Hub der Kolbenvorrichtung (110), um die Kolbenvorrichtung (110; 210) in einer Kammer (160) der Anordnung abzudichten, ausgebildet ist.

8. Medizinische Vorrichtungsanordnung nach Anspruch 7, wobei das Entlüftungsvorrichtungssystem (100; 200; 800) beweglich in verschiedene Teile des Bodens (140) der Kolbenvorrichtung (110; 210) eingreift.

9. Medizinische Vorrichtungsanordnung nach Anspruch 7, wobei das Entlüftungsvorrichtungssystem (100; 200; 800) beweglich um den Boden (140; 240; 810) der Kolbenvorrichtung (110; 210) sitzt.

10. Verfahren zur Verringerung von unerwünschtem Druck in einer Kolbenvorrichtungsanordnung, umfassend die folgenden Schritte:
Entlüften von Luft von oberhalb einer Kolbenvorrichtung (110; 210) um einen Boden (140; 240; 810) der Kolbenvorrichtung (110; 210) bei einem Rückhub mit einem Entlüftungseinrichtungssystem (100; 200; 800) nach Anspruch 1, wobei die Dichtung (120; 220; 820) des Entlüftungsvorrichtungssystems (100; 200; 800) unterhalb des Bodens (140; 240; 810) der Kolbenvorrichtung (110; 210) angeordnet ist; und
Abdichten von Luft unterhalb des Bodens (140; 240; 810) der Kolbenvorrichtung (110; 210) bei einem druckerzeugenden Hub mit der Dichtung (120; 220; 820) des Entlüftungsvorrichtungssystems (100; 200; 800) bei einem druckerzeugenden Hub.

11. Verfahren nach Anspruch 10, ferner umfassend den Schritt des Ausbildens der Dichtung (120; 220; 820) des Entlüftungsvorrichtungssystems (100; 200; 800) zum Bewegen von der zweiten Position (B) im Wesentlichen nahe der Oberseite (140; 240; 810) der Kolbenvorrichtung (110; 210) beim druckerzeugenden Hub in die erste Position (A), von der zweiten Position (B) weg verschoben, und direkt oder indirekt gegen eine untere Fläche der Kolbenbewegung (110; 210) bei einem Rückhub angeordnet.

## Revendications

1. Système à dispositif de ventilation configuré pour permettre à un fluide de s'écouler en direction opposée à un dispositif de va-et-vient (110 ; 210) du fait du mouvement du dispositif de va-et-vient (110 ; 210) lors d'une course de retour, dans lequel le dispositif de va-et-vient (110 ; 210) est un piston et comprend une tête (140 ; 240 ; 810) qui est mobile en va-et-vient dans une chambre (160 ; 260),
le système à dispositif de ventilation comprenant un joint (120 ; 220 ; 820) configuré et agencé pour s'asseoir dans une première position (A), sous la tête (140 ; 240 ; 810) du dispositif de va-et-vient (110 ; 210), durant une course de retour du dispositif de va-et-vient (110 ; 210), dans lequel, dans la première position (A), le joint (120 ; 220 ; 820) procure un chemin de fluide d'une hauteur présélectionnée (h) entre une partie supérieure du joint (120 ; 220 ; 820) et une surface inférieure de la tête (140 ; 240 ; 810) du dispositif de va-et-vient (110 ; 210), et pour s'asseoir dans une deuxième position (B), différente de la première position (A), durant une course de génération de pression ;
dans lequel le joint (120 ; 220 ; 820) est constitué d'un matériau élastique et est en outre configuré pour se déplacer au moins partiellement dans la deuxième position (B) et pour s'étendre, depuis la deuxième position (B), vers une paroi intérieure (265) de la chambre (160 ; 260) pour sceller substantiellement ou complètement le fluide sous la tête (140 ; 240 ; 810) du dispositif de va-et-vient (110 ; 210) durant la course de génération de pression du dispositif de va-et-vient (110 ; 210).

2. Système à dispositif de ventilation selon la revendication 1, dans lequel le système à dispositif de ventilation comprend une tête de piston ventilée (720) avec une rainure et au moins une portion de joint déformable (710) qui ne se déplace que partiellement en réponse au mouvement de la tête de piston ventilée (720).

3. Ensemble tire-lait comprenant :
un système à dispositif de ventilation (100 ; 200 ; 800) selon la revendication 1,
dans lequel le joint (120 ; 220 ; 820) peut être connecté sélectivement à la tête du dispositif de va-et-vient (110 ; 210), et est configuré pour se déplacer sélectivement, sur la base des forces présentes dans le système durant le fonctionnement du dispositif de va-et-vient (110 ; 210).

4. Ensemble tire-lait selon la revendication 3, **le** système à dispositif de ventilation étant configuré pour sceller le dispositif de va-et-vient (110 ; 210) durant une course de génération de pression et pour ventiler l'ensemble durant une course de retour.

5. Ensemble tire-lait selon la revendication 3, comprenant en outre une chambre (160), dans lequel le système à dispositif de ventilation élimine en outre la pression indésirable dans la chambre (160) de l'ensemble durant le fonctionnement sans affecter la création de pression dans la chambre (160) durant une course de génération de pression de l'ensemble.

6. Ensemble tire-lait selon la revendication 3, le système à dispositif de ventilation étant en outre configuré à partir d'un matériau scellable.

7. Ensemble de dispositif médical comprenant :
un système à dispositif de ventilation (100 ; 200 ; 800) selon la revendication 1,
dans lequel le joint (120 ; 220 ; 820) du système à dispositif de ventilation (100 ; 200 ; 800) est configuré pour se déplacer sur la base des forces présentes dans l'ensemble, et est configuré en outre pour s'engager sélectivement avec une portion de la tête du dispositif de va-et-vient (110 ; 210) sous la partie supérieure du dispositif de va-et-vient (110 ; 210) lors d'une course de génération de pression du dispositif de va-et-vient (110) pour sceller substantiellement le dispositif de va-et-vient (110 ; 210) à l'intérieur d'une chambre (160) de l'ensemble.

8. Ensemble de dispositif médical selon la revendication 7, dans lequel le système à dispositif de ventilation (100 ; 200 ; 800) s'engage de manière mobile avec différentes parties de la tête (140) du dispositif de va-et-vient (110 ; 210).

9. Ensemble de dispositif médical selon la revendication 7, le système à dispositif de ventilation (100 ; 200 ; 800) étant assis de manière mobile autour de la tête (140 ; 240 ; 810) du dispositif de va-et-vient (110 ; 210).

10. Procédé de réduction d'une pression indésirable dans un ensemble de dispositif de va-et-vient, comprenant les étapes suivantes :
ventilation de l'air par le dessus d'un dispositif de va-et-vient (110 ; 210) autour d'une tête (140 ; 240 ; 810) du dispositif de va-et-vient (110 ; 210) lors d'une course de retour avec un système à dispositif de ventilation (100 ; 200 ; 800) selon la revendication 1, le joint (120 ; 220 ; 820) du système à dispositif de ventilation (100 ; 200 ; 800) étant disposé sous la tête (140 ; 240 ; 810) du dispositif de va-et-vient (110 ; 210) ; et
scellement de l'air sous la tête (140 ; 240 ; 810) du dispositif de va-et-vient (110 ; 210) lors d'une course de génération de pression avec le joint (120 ; 220 ; 820) du système à dispositif de ventilation (100 ; 200 ; 800) lors d'une course de génération de pression.

11. Procédé selon la revendication 10, comprenant en outre une étape consistant à configurer le joint (120 ; 220 ; 820) du système à dispositif de ventilation (100 ; 200 ; 800) pour qu'il se déplace depuis la deuxième position (B) substantiellement proche de la partie supérieure (140 ; 240 ; 810) du dispositif de va-et-vient (110 ; 210) lors de la course de génération de pression jusqu'à la première position (A), espacée de la deuxième position (B) et disposée directement ou indirectement contre une surface inférieure du dispositif de va-et-vient (110 ; 210) lors d'une course de retour.
